(19) Europäisches Patentamt
European Patent Office
Office européen des brevets

(11) **EP 1 847 215 A1**

(12) **EUROPEAN PATENT APPLICATION**

(43) Date of publication:
**24.10.2007 Bulletin 2007/43**

(51) Int Cl.:
***A61B 5/00*** *(2006.01)*

(21) Application number: **07006887.9**

(22) Date of filing: **02.04.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **21.04.2006 JP 2006117402**

(71) Applicant: **Hitachi, Ltd.**
**Chiyoda-ku**
**Tokyo 100-8280 (JP)**

(72) Inventors:
• **Tanaka, Naoki**
**Tokyo 100-8220 (JP)**
• **Maki, Atsushi**
**Tokyo 100-8220 (JP)**
• **Katsura, Takushige**
**Tokyo 100-8220 (JP)**

(74) Representative: **Strehl Schübel-Hopf & Partner**
**Maximilianstrasse 54**
**80538 München (DE)**

(54) **Living body measurement system and method**

(57) A living body measurement system including a cerebral blood volume measurement unit (120) for measuring a regional cerebral blood volume of a subject, an arterial pressure/heart rate measurement unit (130) for measuring an arterial pressure and/or a heart rate of the subject, an analysis unit (112) for analyzing signals measured by the cerebral blood volume measurement unit and the arterial pressure/heart rate measurement unit, an extraction unit (112) for extracting information concerning a regional cerebral vascular state of the subject on the basis of an output of the analysis unit, and a display unit (113) for displaying a measurement result measured by the cerebral blood volume measurement unit and/or the arterial pressure/heart rate measurement unit, an analysis result analyzed by the analysis unit, or an extraction result extracted by the extraction unit.

FIG. 1

**Description**

BACKGROUND OF THE INVENTION

The present invention relates to a measurement system and method for measuring living bodies to conduct noninvasive cerebrovascular disease tests.

The cerebrovascular disease (or stroke) is a general term for morbid states which bring about neural symptoms by an organic abnormality or a functional abnormality, and it holds the third place in death causes of the Japanese people at the present time. Necrosis (softening) of the brain tissue caused by cerebral infarction, i.e., ischemia occupies 70% of cerebrovascular diseases. In the necrosis (softening) of the brain tissue, there are cerebral thrombosis caused by atherosclerosis of the cerebral artery and cerebral embolism caused by an embolus coming from the outside of the skull. In the hemorrhage, there are cerebral hemorrhage into the cerebral parenchyma and subarachnoid hemorrhage into the subarachnoid space. Tests in the acute stage are conducted by using a blood test, an electrocardiogram, and the X-ray CT, and so on. The MRI, PET, SPECT and cerebrovascular imaging are used subsidiarily.

The regional cerebral blood volume change can be measured in a noninvasive manner by using the optical topography method. The optical topography method is a method of exposing a subject to light having a wavelength belonging to the visible region to the infrared region, detecting light beams of a plurality of signals by using the same photodetector, and measuring the hemoglobin change quantity (JP-A-9-019408 and so on). This method has a feature that the restraint upon the subject is also low as compared with the cerebral function measurement techniques such as the MRI and PET.

List of background technique documents

Patent document 1: JP-A-9-019408
Non-patent document 1: A. Maki et al., Medical Physics, vol. 22, pp. 1997-2005 (1995)
Non-patent document 2: M. L. Schroeter et al., Journal of Cerebral Blood Flow & Metabolism, vol. 24, pp. 1183-1191 (2004)
Non-patent document 3: M. L. Schroeter et al., Journal of Cerebral Blood Flow & Metabolism, vol. 25, pp. 1675-1684 (2005)

In the above-described cerebrovascular disease tests, however, an invasive method is used or a severe burden is cast upon the subject although the test is noninvasive, in some cases. Furthermore, the possibility of overlooking a small lesion is high. Therefore, it is difficult to preventively conduct a cerebrovascular disease test.

SUMMARY OF THE INVENTION

In accordance with the present invention, the cerebral blood volume variation and the arterial pressure and/or the heart rate variation are measured, the cerebral vascular stiffness and their changes are evaluated on the basis of properties of low frequency components in those variations, and a disease region and a danger region are presumed and displayed.

The problem can be solved by a living body measurement system including a cerebral blood volume measurement unit for measuring a regional cerebral blood volume of a subject, an arterial pressure/heart rate measurement unit for measuring an arterial pressure and/or a heart rate of the subject, an analysis unit for analyzing signals measured by the cerebral blood volume measurement unit and the arterial pressure/heart rate measurement unit, an extraction unit for extracting information concerning a regional cerebral vascular state of the subject on the basis of an output of the analysis unit, and a display unit for displaying a measurement result measured by the cerebral blood volume measurement unit and/or the arterial pressure/heart rate measurement unit, an analysis result analyzed by the analysis unit, or an extraction result extracted by the extraction unit.

Hereafter, contents of the present invention will be described in detail with reference to concrete examples.

According to the present invention, it becomes possible to test the cerebrovascular disease in a noninvasive manner and with high precision.

Other objects, features and advantages of the invention will become apparent from the following description of the embodiments of the invention taken in conjunction with the accompanying drawings.

BRIEF DESCRIPTION OF THE DRAWINGS

Fig. 1 shows a configuration of a living body measurement system;
Fig. 2 shows an input screen of the living body measurement system;
Fig. 3 shows an example of a measurement probe;
Figs. 4A and 4B show low frequency components of a power spectrum;
Fig. 5 shows a result display example;
Fig. 6 shows a processing flow in an analysis unit and an extraction unit;
Fig. 7 is a standard vascularity diagram;
Fig. 8 shows result display with sensitivity distribution information attached;
Fig. 9 shows an adjustment procedure for thresholds TH1 and TH2; and
Fig. 10 shows utilization of history information of the same subject.

DESCRIPTION OF THE EMBODIMENTS

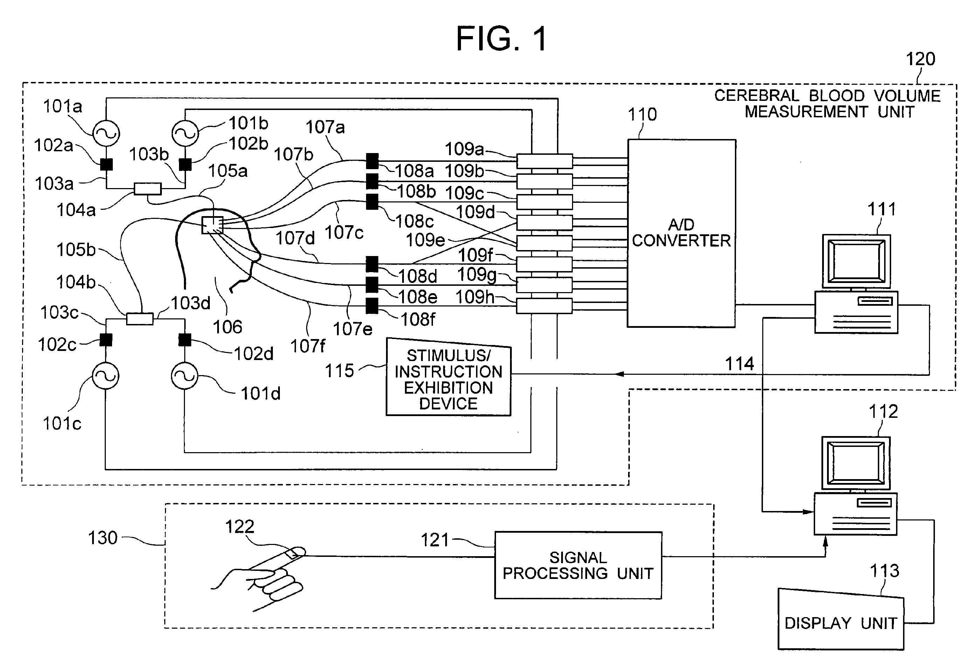

(1) FIG. 1 shows a configuration of a system. The present system includes an input unit, an analysis unit, a storage unit and an extraction unit included in a computer 112, a cerebral blood volume measurement unit 120, an arterial pressure/heart rate measurement unit 130 and a display unit 113. If the computer 112 has the display function, the computer 112 can be substituted for the display unit 113.

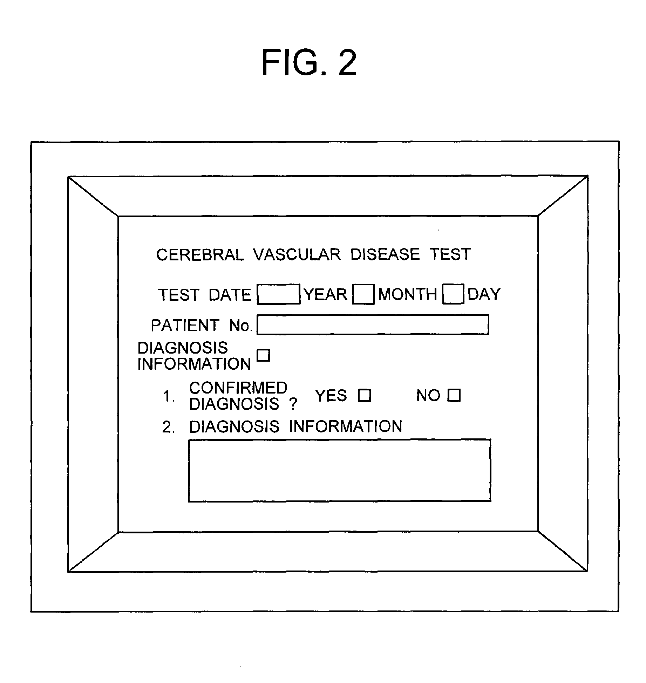

In the input unit, information required for the test, for example, the age, the distinction of sex and the handedness of the subject, diagnosis information such as a diagnosis at the current time point and treatment history, in particular, whether there is a confirmed diagnosis result are input by an operator. How an input screen is displayed is shown in FIG. 2. In this example, the subject is identified by using a patient No. However, the name may be used. If there is a confirmed diagnosis result, a square in item No. 1 is checked. In that case, test results are stored in a database automatically.

The regional cerebral blood volume (oxyhemoglobin, deoxyhemoglobin, and total hemoglobin) is obtained in the cerebral blood volume measurement unit 120 by exposing a head of the subject to light having a wavelength belonging to the visible region to the infrared region, and detecting and measuring light beams of a plurality of signals passed through the inside of the subject by using the same photodetector.

A plurality of light sources 102a to 102d have wavelengths that are different from each other. (The light sources 102a and 102c have a wavelength of 780 nm, and the light sources 102b and 102d have a wavelength of 830 nm.) Modulators conduct strength modulation on light beams emitted from the light sources 102a and 102b (102c and 102d) by using oscillators 101a and 101b (101c and 101d) having different frequencies. A coupler 104a (104b) couples light beams subjected to strength modulation, through optical fibers 103a and 103b (103c and 103d). A plurality of light irradiation means applies a light beam from the coupler 104a (104b) to different positions on a scalp of a subject 106 via a light irradiation optical fiber 105a (105b). A plurality of light sensing optical fibers 107a to 107f are provided so as to have tips positioned in the vicinity of light irradiation positions of the light irradiation means and at equal distances (which are supposed to be 30 mm here) from the light irradiation means. A plurality of light sensing means formed of light sensors 108a to 108f are provided respectively for the light sensing optical fibers 107a to 107f. Light beams transmitted through the living body are collected by using the six light sensing optical fibers 107a to 107f, and subjected to photoelectric conversion in the light sensors 108a to 108f. The light sensing means detect light beams reflected within the subject and convert them to electric signals. As the light sensors 108, photoelectric conversion elements represented by photomultipliers or photodiodes are used.

The electric signals (hereafter referred to as living body passed light strength signals) representing living body passed light strength obtained by photoelectric conversion conducted in the light sensors 108a to 108f are input to lock-in amplifiers 109a to 109h. The light sensors 108c and 108d detect the living body passed light strength collected by the light sensing optical fibers 107c and 107d located at equal distances from the light irradiation optical fibers 105a and 105b. Therefore, each of signals from the light sensors 108c and 108d is separated into two systems. Thus, the signal from the light sensor 108c is input to the lock-in amplifiers 109c and 109e, and the signal from the light sensor 108d is input to the lock-in amplifiers 109d and 109f. Strength modulation frequencies from the oscillators 101a and 101b are input to the lock-in amplifiers 109a to 109d as reference frequencies. Strength modulation frequencies from the oscillators 101c and 101d are input to the lock-in amplifiers 109e to 109h as reference frequencies. Therefore, living body passed light strength signals for the light sources 102a and 102b are separated and output from the lock-in amplifiers 109a to 109d, and living body passed light strength signals for the light sources 102c and 102d are separated and output from the lock-in amplifiers 109e to 109h.

The separated passed light strength signals for respective wavelengths output from the lock-in amplifiers 109a to 109h are subjected to analog-digital conversion in an analog-digital converter 110, and resultant digital signals are sent to a measurement control computer 111. The measurement control computer 111 computes relative change quantities of the oxyhemoglobin concentration, deoxyhemoglobin concentration, and total hemoglobin concentration from signals detected at the detection points according to the procedure described in the non-patent document 1 by using the passed light intensity signals, and stores them in the storage unit as information at the plurality of measurement points with time.

Herein an embodiment in which a plurality of light beams are separated by using the modulation method has been described. However, this is not restrictive, but, for example, a time division method of discriminating a plurality of light beams by shifting timing of applying a plurality of light beams in time can also be used.

On the other hand, the arterial pressure/heart rate measurement unit 130 measures the heart rate and/or the arterial pressure by using the photoplethysmography. A cuff 122 is attached to a finger tip, and the heart rate is detected by optical detection and the arterial pressure (systolic arterial pressure, diastolic arterial pressure, and average arterial pressure) are detected simultaneously. The arterial pressure value is interpolated at a sampling frequency of 200 Hz in a signal processing unit 121, and sent to the analysis unit in the computer 112. Herein, the arterial pressure/heart rate measurement unit 130 for measuring the heart rate and the arterial pressure is shown. However, it is sufficient if either the heart rate or the arterial pressure can be measured. Herein, the photoelectric volume pulse wave recording is used because of easiness of handling. However, it is also possible to use an electrocardiograph for the hear rate and use an invasive sphygmomanometer for the arterial pressure.

The analysis unit analyzes the power spectra of the measured regional cerebral blood volume and the arterial pressure or heart rate. Results of them are delivered to the storage unit in the computer 112.

The storage unit temporarily stores the measurement information on the subject to make subsequent processing possible. On the other hand, for example, if there is a confirmed diagnosis, it is also possible to store the measured information as a database. The database information is used not only when the parameter automatic adjustment is conducted as described later, but also when making a diagnosis on the basis of results of the test conducted by the present system.

The extraction unit in the computer 112 extracts information concerning the cerebrovascular disease from the power spectrum of the signal analyzed by the analysis unit and quantitative information concerning the power spectrum according to a method described later. The information concerning the cerebrovascular disease extracted by the extraction unit is displayed by the display unit 113.

In FIG. 1, the computer 111 and the computer 112 are drawn separately. However, it is a matter of course that one computer may be used instead of the computer 111 and the computer 112.

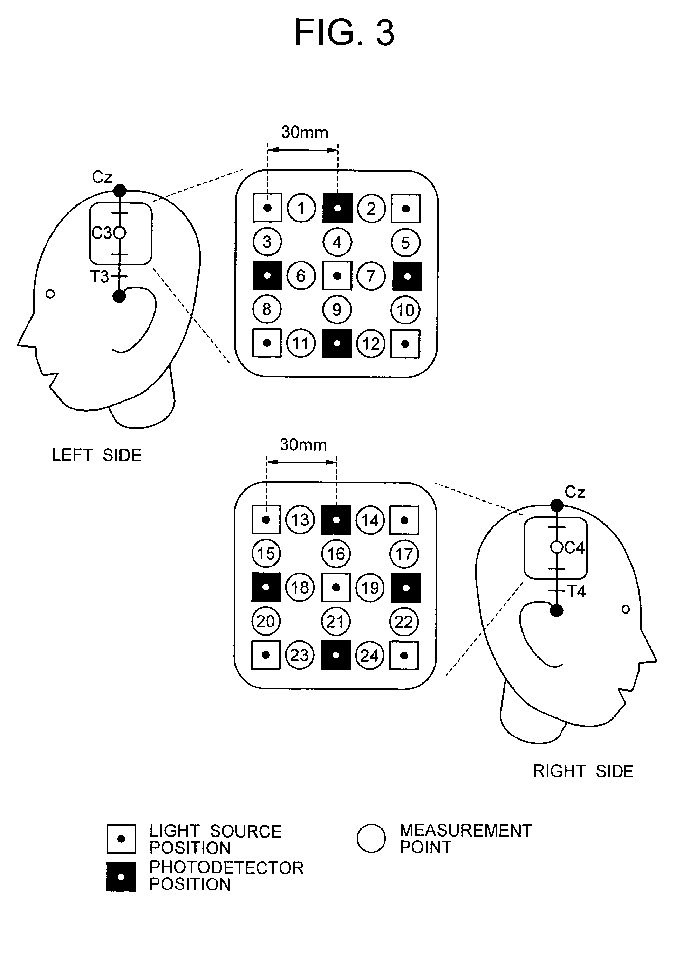

FIG. 3 schematically shows a probe for measuring the cerebral blood volume. Cz, T3 and T4 are characters indicating standard positions for electroencephalogram measurement, and represent the vertex, a part located immediately above the left ear, and a part located immediately above the right ear, respectively. C3 and C4 denote the middle point between Cz and T3 and the middle point between Cz and T4, respectively. Measurement is possible for 12 left channels and 12 right channels, i.e., 24 channels in total. Each channel is identified by a number (hereafter referred to as channel number) given to a measurement point.

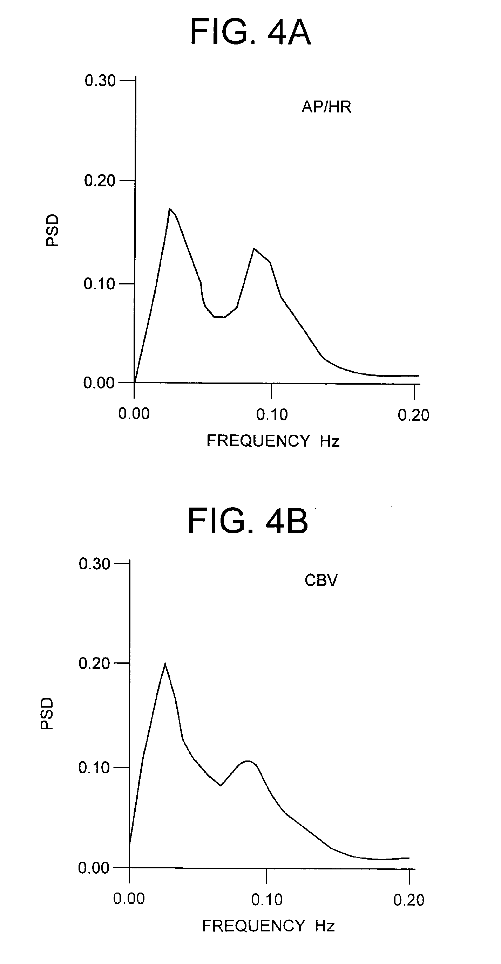

Examples of the power spectrum obtained by the analysis unit are shown in Figs. 4A and 4B. Fig. 4A shows an average value (represented as AP/HR) of a power spectrum obtained from the arterial pressure (AP) and heart rate (HR). Fig. 4B shows a power spectrum of the regional cerebral blood volume (CBV). The ordinate represents the power spectral density (PSD). The power spectral density is normalized so as to yield unity when power is integrated with respect to the frequency. The frequency on the abscissa is a coordinate corresponding to a time axis obtained by conducting Fourier transform on the arterial pressure & heart rate which is time series data, and includes a range from zero Hz to the Nyquist frequency (half of the sampling frequency). Figs. 4A and 4B show a part thereof. The arterial pressure & heart rate shown in Fig. 4A has been measured by attaching a probe to the third finger of the left hand and using the arterial pressure/heart rate measurement unit 130. A result shown in Fig. 4B has been calculated from the regional cerebral blood volume measured on the channel 4 in Fig. 3. The arterial pressure & heart rate is compared with the regional cerebral blood volume with respect to a power ratio $R_p$. Here, the power ratio $R_p$ is represented as $R_p = P_{LF}/P_{VLF}$ and it is the ratio of average power $P_{LF}$ in a low-frequency (LF) region (0.07-0.11 Hz) to average power $P_{VLF}$ in a very-low-frequency (VLF) region (0.01-0.05 Hz). As a result, it is appreciated that the arterial pressure & heart rate is greater in power ratio $R_p$ than the regional cerebral blood volume. Fluctuations in these regions deeply relate to the regulation function of the vascular system. The regulation is dominated by a vasomotor center, a sympathetic nerve, a parasympathetic nerve (vagus nerve) or the like. However, the fluctuations are partly neurogenic and partly myogenic. According to the non-patent documents 2 and 3 written by Schroeter etc., the LF region component of the cerebral blood volume is weakened by aging, whereas a large change is not noticed in the VLF region component, and both the LF region component and the VLF region component of the cerebral blood volume are weakened by the microangropathy, but the LF region component is weakened more violently. Changes caused by aging have deep relations to the fiberization of a smooth muscle, and changes caused by the microangropathy have deep relations to an occlusion and sclerosis phenomenon caused in a thin small blood vessel by a thrombus. Any change is considered to have been caused by degeneration in a smooth muscle. The power ratio $R_p$ tends to be decreased in value by either of aging and the microangropathy. In other words, it can be said that fluctuations in the LF region are more myogenic than those in the VLF region. Results in Figs. 4A and 4B indicate that on average the blood vessel in the finger tip is softer as to the vascular stiffness.

In the present invention, the power ratio $R_p$, which is the ratio of the average power $P_{LF}$ in the LF region (0.07-0.11 Hz) to the average power $P_{VLF}$ in the VLF region (0.01-0.05 Hz), is used as an index for the vascular stiffness. For the above-described reason, the power ratio $R_p$ becomes a parameter reflecting the vascular stiffness. As described above, the power ratio $R_p$ is calculated on the basis of the average power values in respective regions. Or the power ratio $R_p$ may be calculated from a ratio between power integral values in corresponding frequency regions. In addition, if the 1/f spectrum component in the power spectrum is previously removed at that time, the vascular property can be evaluated more accurately. The 1/f spectrum component is a spectrum structure that does not have a characteristic frequency and that is often observed in the power spectrum of a living body, especially the artery pressure & heart rate. It is considered that the 1/f spectrum component represents that the variabilities in artery pressure and heart rate are generated from a complicated feedback structure. However, details of the generation mechanism are still unknown.

The method for previously removing the 1/f spectrum component will be described hereafter. First, the power spectrum is plotted in full logarithmic exhibition, and the low frequency part is approximated by a straight line.

[Expression 1]

$$\log P(f) = -\alpha \log f + \beta$$

A slope $\alpha$ represents an exponent of the 1/f spectrum component. These parameters $\alpha$ and $\beta$ are determined by using the least square method, and a spectrum with the straight line part removed is calculated. When conducting the least square calculation, the data of the LF region and the VLF region are not included. In Figs. 4A and 4B, this processing has been conducted.

The vessel in that region is judged to be stiffer as the power ratio $R_p$ thus found becomes smaller. In addition, power ratios respectively obtained from the arterial pressure & heart rate and the regional cerebral blood volume are denoted by $R_p(AP/HR)$ and $R_p(CBV)$, respectively. Hereafter, AP, HR and CBV in a parenthesis represent a relation to the artery pressure, the heart rate and the regional cerebral blood volume, respectively. As for the arterial pressure & heart rate, a power spectrum is found from each of the arterial pressure and the heart rate independently. An average of resultant power spectra is found and regarded as a power spectrum of the arterial pressure & heart rate. The power ratio $R_p$ is calculated on the basis of the averaged power spectrum. Instead of using both the arterial pressure and the heart rate, only either the arterial pressure or the heart rate may be used. At that time, indication AP or HR is used so as to correspond to actually used data, instead of the indication AP/HR. The power ratios obtained from the arterial pressure & heart rate and the regional cerebral blood volume are represented by the following expressions.

[Expression 2]

$$R_p(AP/HR) = P_{LF}(AP/HR)/P_{VLF}(AP/HR)$$

[Expression 3]

$$R_{\mathrm{p}}(CBV) = P_{\mathrm{LF}}(CBV)/P_{\mathrm{VLF}}(CBV)$$

In the present embodiment, the test for the vascular disease is conducted by using these relations (the expressions 2 and 3) and the following two criteria.

   <1> When

[Expression 4]

$$R_{\mathrm{p}}(AP/HR) < TH1$$

   or

[Expression 5]

$$R_{\mathrm{p}}(CBV) < TH1,$$

a cerebral vascular disease tends to occur.
   <2> In a region satisfying the (expression 4 or 5) and

[Expression 6]

$$R_{\mathrm{p}}(CBV)/R_{\mathrm{p}}(AP/HR) < TH2,$$

a cerebral vascular disease especially tends to occur.

The extraction unit extracts information concerning the cerebral vascular disease in accordance with the two criteria. The threshold TH1 is set equal to 0.1. This is determined on the basis of a preliminary study conducted on subjects that are medically sufficient in number. Automatic parameter adjustment based on some data of a confirmed diagnosis will be described later. The criterion <1> detects general vascular stiffness. Although it is difficult to determine which of the genetic condition and living custom causes the stiffness, it is guessed that a subject satisfying this criterion is in a state in which a cerebral vascular disease is apt to occur. On the other hand, in the criterion <2>, a preventive diagnosis of the cerebral vascular disease is conducted paying attention to an especially small power ratio which is seen in the cerebral blood volumes of some subjects. A very great deal of blood is circulating through the brain as compared with other organs. It is considered that this is apt to cause the sclerosis phenomenon of the cerebrovascular system and this relates to an especially small power ratio seen in the cerebral blood volume. The threshold is set equal to TH2=0.2, and this criterion is regarded as being satisfied when the power ratio found by using the arterial pressure and/or the heart rate is at least five times large. This value has also been determined on the basis of a preliminary study conducted on a comparatively small number of subjects. If data of confirmed diagnoses are stored as database in the future, improvement can be made by the automatic parameter adjustment function described later.

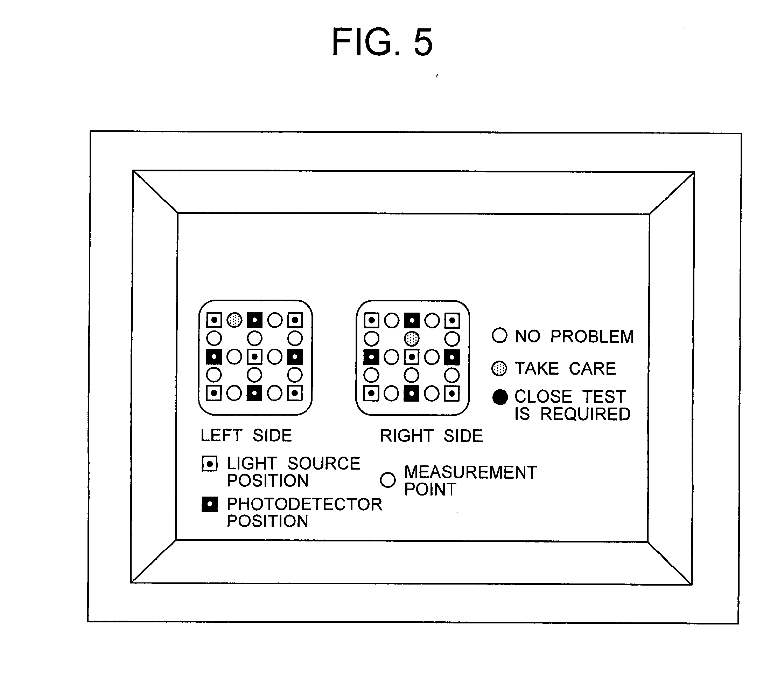

In the present embodiment, one left channel and one right channel satisfy the criterion <1>. The display unit displays the results as shown in Fig. 5. Here, the risk of the cerebral vascular disease is displayed in the following three stages. A first stage is displayed with white to indicate that there is no problem. A second stage is displayed with gray to indicate that care must be taken and the progress should be observed. A third stage is displayed with black to indicate that a close test is required. In this example, a great part is in the first stage and only two channels are in the second stage.

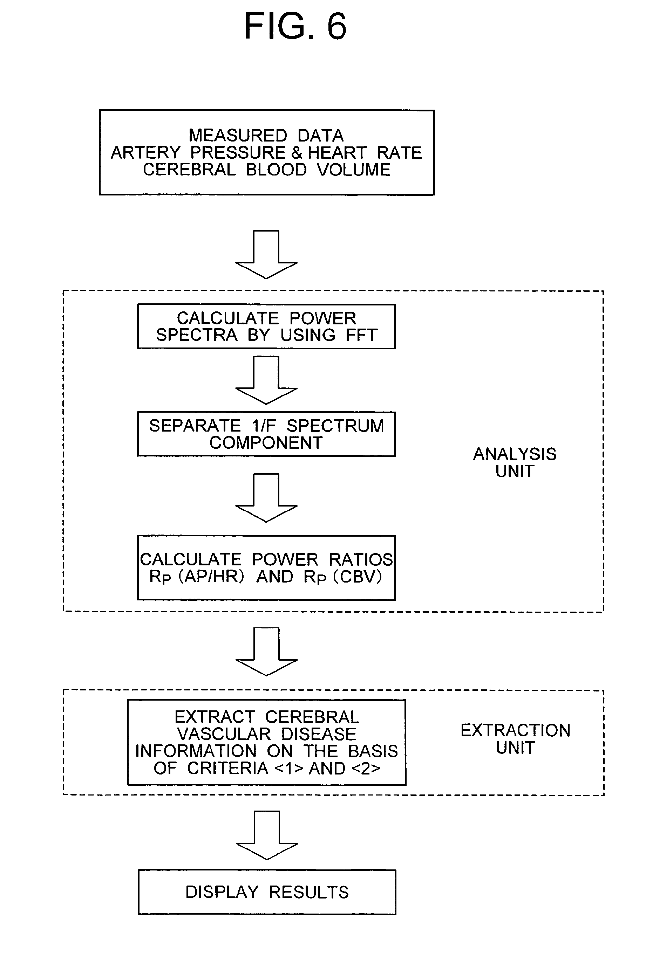

Fig. 6 shows a flow of processing conducted especially in the analysis unit and the extraction unit ranging from the acquisition of measured data to the result display. The power spectra are calculated from the measured data of the cerebral blood volume and the arterial pressure or the heart rate by using the Fast Fourier Transform (FFT). As for the power spectrum calculation method, there are various methods such as nonparametric methods like the Welch method and parametric methods like the Yule method. Any of these methods may be used. Subsequently, the 1/f spectrum component is removed from the obtained power spectra by using the above-described method, and the power ratios $R_P$(AP/HR) and $R_P$(CBV) are calculated according to the above-described method. The extraction unit extracts information concerning the cerebral vascular disease on the basis of the obtained power ratios and the criteria <1> and <2>. The display unit displays results.

(2) However, these results are susceptible to the influence of the vascularity. It is important to conduct an analysis with the influence of the vascularity taken into consideration. Hereafter, a measurement method with the influence of the vascularity taken into consideration will be described.

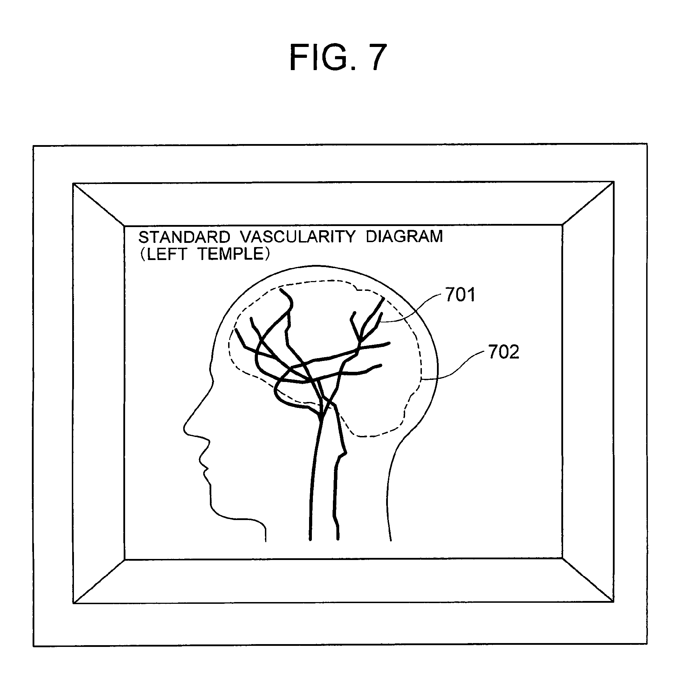

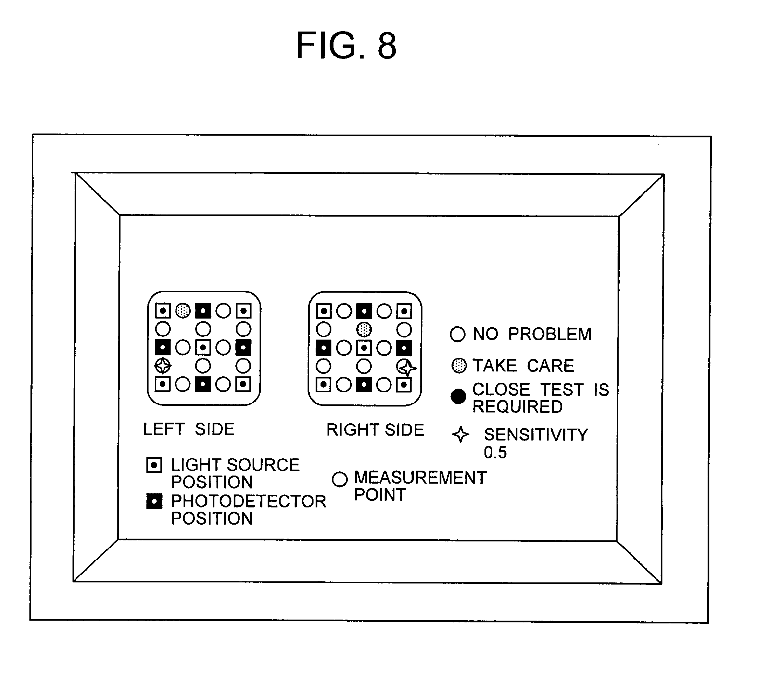

First, a sensitivity map is created according to the measurement regions from a standard vascularity diagram previously stored in the storage unit. An example of the standard vascularity diagram is shown in Fig. 7. A dotted line roughly represents the distribution of the cerebral tissue. Here, arterial distribution is represented by thick solid lines. The anterior cerebral artery, the middle cerebral artery and their terminal branches are included. The standard vascularity diagram is bisymmetrical. The standard vascularity diagram is prepared for the frontal, the parietal and the occipital areas as well besides the temporal area. A procedure for creating a sensitivity map on the basis of the standard vascularity diagram will now be described. In the present embodiment, a sensitivity map of two stages is created. The standard sensitivity is set equal to 1, and the sensitivity is set equal to 0.5 in a region where a comparatively thick artery is located. The power ratio $R_p$ appearing in the expression 2 or 3 is corrected with the sensitivity. The correction is conducted by dividing the measured power ratio by the sensitivity. According to the non-patent documents 2 and 3, a motion of a thickish blood vessel is apt to appear in the VLF region strongly, and the VLF region variation is nerval motion and is hardly susceptible to the influence of aging and the microangropathy. The above-described correction has been determined by taking such facts into consideration. Especially thick blood vessels show a marked trend toward absorption of the light beam, and it is necessary to conduct measurements while avoiding positions of them as far as possible. It is possible in the display unit 113 to display sensitivity distribution obtained from the standard vascularity information together. In the case of the present embodiment, the display becomes as shown in Fig. 8. It is indicated that the sensitivity becomes 0.5 at channel Nos. 8 and 22.

In a region having a thick blood vessel, detected light tends to become weak, and consequently it can be denied that the reliability becomes low. Therefore, such information is information that is useful to the user.

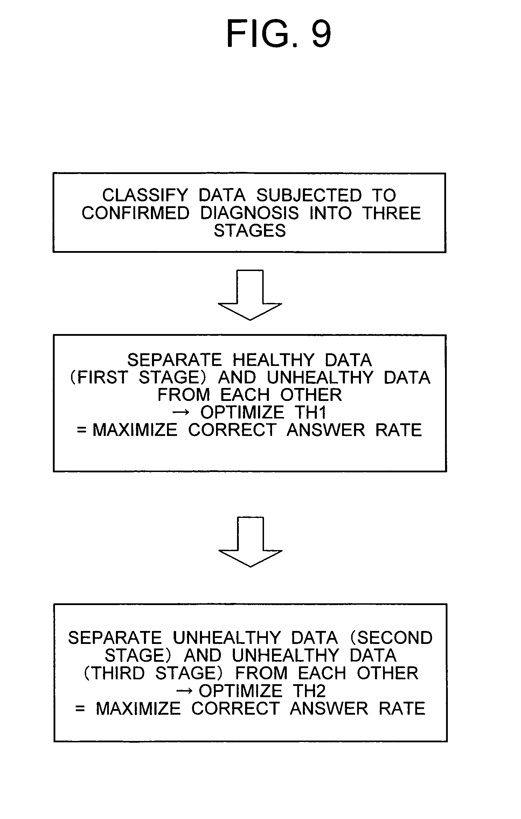

(3) The thresholds used in extraction of the information concerning the cerebral vascular disease can be improved by storing data. The present system has an automatic adjustment function for that purpose. A threshold adjustment procedure is summarized in Fig. 9. First, channels of data obtained from a confirmed diagnosis are classified into three stages: "no problem"; "care must be taken (progress should be observed)"; and "close test is required". The threshold TH1 is optimized on the basis of the information of the power ratio stored in the storage unit. TH1 is a threshold for separating the first stage from the rest. TH1 is determined so as to maximize the average of the correct answer rates in respective groups. The threshold TH2 is optimized. TH2 is a threshold for separating the second stage from the third stage. TH2 is determined so as to maximize the average of the correct answer rates in respective groups. It is possible to change the thresholds to more suitable values by thus conforming to the confirmed diagnosis information.

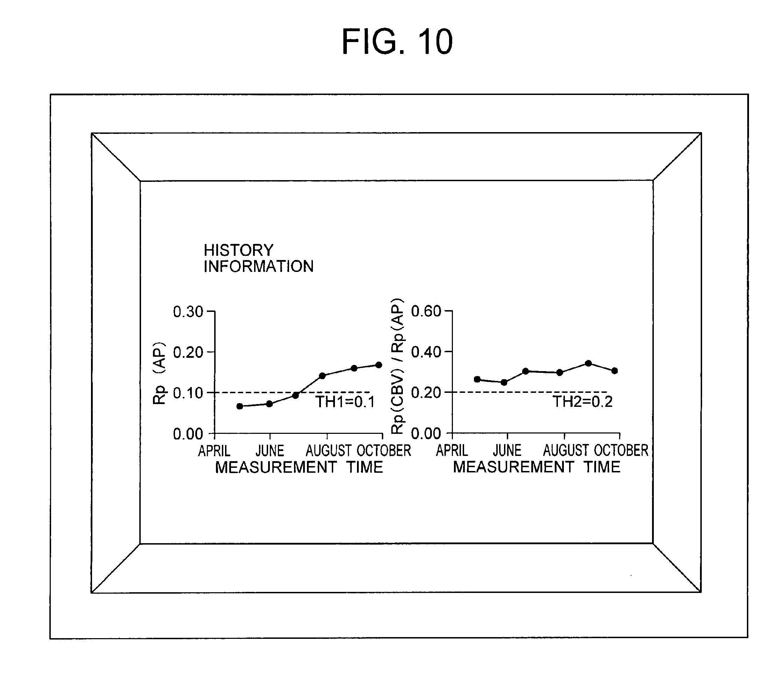

(4) In the present system, it is possible to provide information useful to the diagnosis and treatment by suitably displaying serially stored history information such as measured, analyzed and extracted information of the same subject. One example is shown in Fig. 10. Measurements are conducted on the subject once per month for half a year, i.e., six times in total. Until July, the risk is in the second stage (care must be taken). From August on, the risk is in the first stage (no problem). It is considered to be an effect of treatment conducted during that time. It is also possible to evaluate an effect of a specific treatment by thus utilizing the history information.

The present invention can be applied to a measurement system and method for measuring living bodies to conduct noninvasive cerebrovascular disease tests.

It should be further understood by those skilled in the art that although the foregoing description has been made on embodiments of the invention, the invention is not limited thereto and various changes and modifications may be made without departing from the spirit of the invention and the scope of the appended claims.

**Claims**

1. A living body measurement system comprising:

 a cerebral blood volume measurement unit (120) for measuring a regional cerebral blood volume of a subject;
 an arterial pressure/heart rate measurement unit (130) for measuring an arterial pressure and/or a heart rate of the subject;
 an analysis unit (112) for analyzing signals measured by the cerebral blood volume measurement unit and the arterial pressure/heart rate measurement unit;
 an extraction unit (112) for extracting information concerning a regional cerebral vascular state of the subject on the basis of an output of the analysis unit; and
 a display unit (113) for displaying a measurement result measured by the cerebral blood volume measurement unit and/or the arterial pressure/heart rate measurement unit, an analysis result analyzed by the analysis unit, or an extraction result extracted by the extraction unit.

2. The system of claim 1, further comprising an input unit for inputting living body information and/or diagnosis information of the subject.

3. The system of claim 1, wherein the display unit classifies the information concerning the regional cerebral vascular state of the subject into classes for each region in the head.

4. The system of claim 1, wherein the display unit displays history of past diagnosis information of the subject.

5. The system of claim 1, further comprising a storage unit (112) for storing a measurement result measured by the cerebral blood volume measurement unit and/or the arterial pressure/heart rate measurement unit, an analysis result analyzed by the analysis unit, or an extraction result extracted by the extraction unit as a database, wherein the extraction unit extracts the information concerning the regional cerebral vascular state of the subject on the basis of the database stored in the storage unit and an output of the analysis unit.

6. The system of claim 5, wherein the database stores standard cerebral vascularity information, and the extraction unit conducts sensitivity correction in each cerebral region for the subject on the basis of the standard cerebral vascularity information.

7. The system of claim 4, further comprising a storage unit (112) for storing confirmed diagnosis information of a plurality of subjects, wherein the extraction unit determines magnitude of the first threshold and/or the second threshold so as to maximize a correct answer rate of the extraction on the basis of the confirmed diagnosis information.

8. The system of claim 1, wherein the cerebral blood volume measurement unit (120) comprises:

 a plurality of light irradiation means (105) for applying light beams to a head of the subject;
 a plurality of light sensing means (108) for detecting light beams applied from the light irradiation means and passed through a head of the subject; and
 a computing unit (111) for calculating a blood volume change of the head of the subject on the basis of light beams detected by the light sensing means.

9. A living body measurement method comprising the steps of:

 measuring a regional cerebral blood volume of a subject;
 measuring an arterial pressure and/or a heart rate of the subject;
 analyzing the measured regional cerebral blood volume and the measured arterial pressure and/or heart rate; and
 extracting information concerning a regional cerebral vascular state of the subject on the basis of information obtained by the analysis.

10. The system of claim 1 or the method of claim 9, including analyzing power spectra of the measured regional cerebral blood volume and the measured arterial pressure and/or heart rate.

11. The system or method of claim 10, including calculating a first power strength ratio in a first frequency band and a second frequency band of the artery pressure and/or the heart'rate and a second power strength ratio in the first frequency band and the second frequency band of the regional cerebral blood volume on the basis of an analysis result obtained at the analyzing step, and extracting information concerning the cerebral vascular state on the basis of whether the first power strength ratio and/or the second power strength ratio is greater than a first threshold.

12. The system or method of claim 11, including extracting information concerning the cerebral vascular state on the basis of whether the first power strength ratio and the second power strength ratio is greater than a second threshold.

13. The system or method of claim 11, wherein the first frequency band is in a range of 0.01 to 0.05 Hz, and the second frequency band is in a range of 0.07 to 0.11 Hz.

14. The system or method of claim 10, including removing a 1/f spectrum component from the power spectra.

# FIG. 1

CEREBRAL BLOOD VOLUME MEASUREMENT UNIT 120

110 A/D CONVERTER

111

115 STIMULUS/ INSTRUCTION EXHIBITION DEVICE

114

112

130

122

121 SIGNAL PROCESSING UNIT

113 DISPLAY UNIT

EP 1 847 215 A1

# FIG. 2

CEREBRAL VASCULAR DISEASE TEST

TEST DATE [____] YEAR [__] MONTH [__] DAY

PATIENT No. [_____]

DIAGNOSIS
INFORMATION □

　1. CONFIRMED
　　 DIAGNOSIS ?　YES □　　　NO □

　2. DIAGNOSIS INFORMATION

[_____]

EP 1 847 215 A1

# FIG. 3

LEFT SIDE

RIGHT SIDE

▫ LIGHT SOURCE POSITION
◼ PHOTODETECTOR POSITION
◯ MEASUREMENT POINT

# FIG. 4A

# FIG. 4B

# FIG. 5

LEFT SIDE          RIGHT SIDE

⊡ LIGHT SOURCE POSITION
◼ PHOTODETECTOR POSITION
○ MEASUREMENT POINT

○ NO PROBLEM
⊛ TAKE CARE
● CLOSE TEST IS REQUIRED

EP 1 847 215 A1

# FIG. 6

```
┌─────────────────────────────────┐
│         MEASURED DATA           │
│ ARTERY PRESSURE & HEART RATE    │
│    CEREBRAL BLOOD VOLUME        │
└─────────────────────────────────┘
```

⬇

```
┌ ─ ─ ─ ─ ─ ─ ─ ─ ─ ─ ─ ─ ─ ─ ─ ─ ─ ─ ─ ─ ┐
│   ┌─────────────────────────┐           │
│   │    CALCULATE POWER      │           │
│   │  SPECTRA BY USING FFT   │           │
│   └─────────────────────────┘           │
│              ⬇                          │
│   ┌─────────────────────────┐           │
│   │   SEPARATE 1/F SPECTRUM │           │
│   │        COMPONENT        │    ANALYSIS│
│   └─────────────────────────┘     UNIT   │
│              ⬇                          │
│   ┌─────────────────────────┐           │
│   │ CALCULATE POWER RATIOS  │           │
│   │ RP (AP/HR) AND RP (CBV) │           │
│   └─────────────────────────┘           │
└ ─ ─ ─ ─ ─ ─ ─ ─ ─ ─ ─ ─ ─ ─ ─ ─ ─ ─ ─ ─ ┘
```

⬇

```
┌ ─ ─ ─ ─ ─ ─ ─ ─ ─ ─ ─ ─ ─ ─ ─ ─ ─ ─ ─ ─ ┐
│   ┌─────────────────────────┐           │
│   │    EXTRACT CEREBRAL     │           │
│   │    VASCULAR DISEASE     │  EXTRACTION│
│   │ INFORMATION ON THE BASIS│    UNIT    │
│   │ OF CRITERIA <1> AND <2> │           │
│   └─────────────────────────┘           │
└ ─ ─ ─ ─ ─ ─ ─ ─ ─ ─ ─ ─ ─ ─ ─ ─ ─ ─ ─ ─ ┘
```

⬇

```
┌─────────────────────────────────┐
│        DISPLAY RESULTS          │
└─────────────────────────────────┘
```

# FIG. 7

STANDARD VASCULARITY DIAGRAM
(LEFT TEMPLE)

701

702

EP 1 847 215 A1

# FIG. 8

LEFT SIDE          RIGHT SIDE

⊡ LIGHT SOURCE
POSITION                    ○ MEASUREMENT
▣ PHOTODETECTOR       POINT
POSITION

○ NO PROBLEM

⊛ TAKE CARE

● CLOSE TEST IS
REQUIRED

✧ SENSITIVITY
0.5

EP 1 847 215 A1

# FIG. 9

CLASSIFY DATA SUBJECTED TO
CONFIRMED DIAGNOSIS INTO THREE
STAGES

SEPARATE HEALTHY DATA
(FIRST STAGE) AND UNHEALTHY DATA
FROM EACH OTHER
→ OPTIMIZE TH1
= MAXIMIZE CORRECT ANSWER RATE

SEPARATE UNHEALTHY DATA (SECOND
STAGE) AND UNHEALTHY DATA
(THIRD STAGE) FROM EACH OTHER
→ OPTIMIZE TH2
= MAXIMIZE CORRECT ANSWER RATE

# FIG. 10

HISTORY
INFORMATION

TH1=0.1

TH2=0.2

EP 1 847 215 A1

**European Patent Office**

## PARTIAL EUROPEAN SEARCH REPORT

which under Rule 45 of the European Patent Convention shall be considered, for the purposes of subsequent proceedings, as the European search report

Application Number

EP 07 00 6887

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/011128 A1 (ORSAN MEDICAL EQUIPMENT LTD [IL]; SHAPIRA AHARON [IL]; RAPPAPORT ALON) 2 February 2006 (2006-02-02) <br> * abstract; figures 1,2,5 * <br> * page 2, line 28 - line 30 * <br> * page 3, line 19 - page 4, line 11 * <br> * page 7, line 1 - line 13 * <br> * page 14, line 29 - page 15, line 6 * <br> * page 16, line 8 - line 25 * | 1,3-5,7 | INV. <br> A61B5/00 |
| Y | | 2,6,8 | |
| Y | EP 1 452 136 A (HITACHI LTD [JP]) <br> 1 September 2004 (2004-09-01) <br> * abstract; figures 1,2,4,8-10 * <br> * paragraphs [0014], [0058] * | 2,8 | |
| Y | US 2004/240612 A1 (SUZUKI KATSUMI [JP]) <br> 2 December 2004 (2004-12-02) <br> * abstract; figure 1 * | 6 | |
| A | EP 0 807 402 A (OHMEDA INC [US]) <br> 19 November 1997 (1997-11-19) <br> * the whole document * | 1-8 | TECHNICAL FIELDS SEARCHED (IPC) <br><br> A61B |

-/--

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 5 September 2007 | Pereda Cubián, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent Office**

**PARTIAL EUROPEAN SEARCH REPORT**

Application Number

EP 07 00 6887

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| A | WO 03/059164 A (ORSAN MEDICAL EQUIPMENT LTD [IL]; BEN-ARI SHLOMI [IL]; RAPPAPORT ALON) 24 July 2003 (2003-07-24) * the whole document * ----- | 1-8 | |
| A | EP 0 502 270 A1 (HAMAMATSU PHOTONICS KK [JP]) 9 September 1992 (1992-09-09) * the whole document * ----- | 1-8 | |
| A | EP 1 380 253 A (HITACHI LTD [JP]; HITACHI MEDICAL CORP [JP]) 14 January 2004 (2004-01-14) * the whole document * ----- | 1-8 | |
| A | US 2006/025688 A1 (HAYASE TOSHIYUKI [JP] ET AL) 2 February 2006 (2006-02-02) * the whole document * ----- | 1-8 | TECHNICAL FIELDS SEARCHED (IPC) |

EPO FORM 1503 03.82 (P04C10)

European Patent
Office

INCOMPLETE SEARCH
SHEET C

Application Number

EP 07 00 6887

Claim(s) not searched:
        9-14

Reason for the limitation of the search (non-patentable invention(s)):

Article 52 (4) EPC - Diagnostic method practised on the human or animal body

        -----

Further limitation of the search

Claim(s) not searched:
        10-14

Reason for the limitation of the search:

Dependent claims 10-14 refer to either an apparatus (system) claim or a method claim, which introduces a doubt in its category. This produces a lack of clarity of the category of the claim (Article 84 EPC).

## ANNEX TO THE EUROPEAN SEARCH REPORT
## ON EUROPEAN PATENT APPLICATION NO.

EP 07 00 6887

This annex lists the patent family members relating to the patent documents cited in the above-mentioned European search report.
The members are as contained in the European Patent Office EDP file on
The European Patent Office is in no way liable for these particulars which are merely given for the purpose of information.

05-09-2007

| Patent document cited in search report | | Publication date | Patent family member(s) | | Publication date |
|---|---|---|---|---|---|
| WO 2006011128 | A1 | 02-02-2006 | EP<br>EP<br>WO | 1796536 A1<br>1786316 A1<br>2006006143 A1 | 20-06-2007<br>23-05-2007<br>19-01-2006 |
| EP 1452136 | A | 01-09-2004 | JP<br>US | 2004261265 A<br>2004171919 A1 | 24-09-2004<br>02-09-2004 |
| US 2004240612 | A1 | 02-12-2004 | CN<br>WO | 1518430 A<br>03000136 A1 | 04-08-2004<br>03-01-2003 |
| EP 0807402 | A | 19-11-1997 | CA<br>JP<br>JP<br>JP<br>US | 2201577 A1<br>10052408 A<br>3350521 B2<br>2001178709 A<br>5766127 A | 15-10-1997<br>24-02-1998<br>25-11-2002<br>03-07-2001<br>16-06-1998 |
| WO 03059164 | A | 24-07-2003 | AU<br>US | 2003209608 A1<br>2005054939 A1 | 30-07-2003<br>10-03-2005 |
| EP 0502270 | A1 | 09-09-1992 | DE<br>DE<br>JP<br>US | 69123954 D1<br>69123954 T2<br>5084233 A<br>5251632 A | 13-02-1997<br>30-04-1997<br>06-04-1993<br>12-10-1993 |
| EP 1380253 | A | 14-01-2004 | JP<br>US | 2004037408 A<br>2004006260 A1 | 05-02-2004<br>08-01-2004 |
| US 2006025688 | A1 | 02-02-2006 | JP | 2004121735 A | 22-04-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9019408 A

**Non-patent literature cited in the description**

- **A. MAKI et al.** *Medical Physics,* 1995, vol. 22, 1997-2005
- **M. L. SCHROETER et al.** *Journal of Cerebral Blood Flow & Metabolism,* 2004, vol. 24, 1183-1191
- **M. L. SCHROETER et al.** *Journal of Cerebral Blood Flow & Metabolism,* 2005, vol. 25, 1675-1684